# EUROPEAN PATENT APPLICATION

(11) **EP 0 553 954 A2**
(43) Date of publication of application: **04.08.1993**
(21) Application number: 93300065.5
(22) Date of filing: 06.01.1993
(51) Int. Cl.: C07C 213/02, C07C 217/08

(54) **Preparation of tetramethyldiamino-polyoxyethylenes**

(30) Priority: 27.01.1992 US 825899
(71) Applicant: Huntsman Corporation, Salt Lake City, Utah 84111-1053 (US)
(72) Inventor: Zimmerman, Robert LeRoy, Austin, Texas 78759 (US)
(74) Representative: Brock, Peter William

(57) **Abstract**

Tetramethyldiamino-polyoxyethylenes having the formula:

(I) (CH₃)₂N(CH₂)₂-O-[(CH₂)₂-O-]_{y}-(CH₂)₂N(CH₃)₂

wherein y is 1 to 2,
can be produced with high yield and selectivity by contacting a diamino-polyoxyethylene having the formula:

(II) H₂N(CH₂)₂-O-[(CH₂)₂-O-]_{y}-(CH₂)₂NH₂

with formaldehyde, at elevated temperature and pressure, in the presence of hydrogen and a nickel or cobalt hydrogenation catalyst, preferably a nickel, copper, chromium catalyst.

## Description

This invention relates to a method for the preparation of tetramethyldiamino-polyoxyethylenes. More particularly, this invention relates to a method for the preparation of tetramethyldiamino-polyoxyethylenes by the reaction of a diamino-polyoxyethylene with formaldehyde in the presence of hydrogen and a hydrogenation catalyst.

Hazard et al., in Bull. Soc. Chem. Fr., 1953 (270-273) have disclosed a process for the preparation of tetramethyldiamino-polyoxyethylenes by the reaction of, for example, 1,2-bis(2-chloroethoxy)ethane and dimethylamine.

US-A-3167551 discloses a process for the preparation of N-methylated heterocyclic compounds, such as N-methyl piperazine, by the reaction of piperazine with excess formaldehyde.

US-A-3249613 discloses a process for the preparation of 1,4-dimethylpiperazine from piperazine, formaldehyde and hydrogen, using a nickel, copper, chromium catalyst.

US-A-3152998 discloses a nickel, copper, chromium catalyst useful in the preparation of heterocyclic nitrogen compounds.

JP-A-63077918 and JP-A-62236815 disclosed the use of compounds such as 2,2'-[1,2-ethanediylbis(oxy)bis-[N,N-dimethylethanamine]] as catalysts for the preparation of polyurethanes.

This invention relates to a method for the preparation from diamino-polyoxyethylenes of tetramethyldiamino-polyoxyethylenes, which are useful as polyurethane catalysts.

It has been surprisingly discovered, in accordance with the present invention, that a substantially quantitative yield of a high purity tetramethyldiamino-polyoxyethylene can be obtained by the reaction described more particularly below.

This invention provides a method for the preparation of a tetramethyldiamino-polyoxyethylene having the formula:

(I) (CH₃)₂N(CH₂)₂-O-[(CH₂)₂-O-]_{y}-(CH₂)₂N(CH₃)₂

by reacting a diamino-polyoxyethylene having the formula:

(II) H₂N(CH₂)₂-O-[(CH₂)₂-O-]_{y}-(CH₂)₂NH₂

wherein y is 1 to 2, with a molar excess of formaldehyde in contact with a nickel or cobalt hydrogenation catalyst and in the presence of hydrogen at a temperature of 100 to 200°C and a pressure of 0.75 to 35.0 MPa.

The starting materials for the present invention are a diamino-polyoxyethylene, formaldehyde, hydrogen and a solvent, preferably one selected from water and methanol.

Representative examples of the diamino polyoxyethylene feedstocks of formula (II) include, for example, triethyleneglycol diamine, a material available from Texaco Chemical Company under the tradename JEFFAMINE EDR-148, and tetraethyleneglycol diamine, a material available from Texaco Chemical Company under the tradename JEFFAMINE EDR-192.

Formaldehyde is the other principal reactant used in accordance with the present invention, and is suitably brought into contact with the diamino-polyoxyethylene and the hydrogenation catalyst in solution in a solvent selected from water and methanol. The formaldehyde solution will suitably contain from 10 to 55 wt.% of formaldehyde.

The hydrogenation catalyst that is used in accordance with the present invention is a nickel catalyst, such as Raney nickel, a cobalt catalyst such as Raney cobalt, or a nickel, copper, chromium catalyst preferably comprising, on an oxygen-free basis, 60 to 85 mol % of nickel, 14 to 37 mol % of copper and 1 to 3 mol % of chromium and, more preferably, from 72 to 78 mol % of nickel, 20 to 25 mol % of copper and 1 to 3 mol % of chromium. The preparation of nickel-copper-chromium catalyst compositions of this nature is disclosed in US-A-3152998.

In accordance with a preferred embodiment of the present invention, the reaction is conducted on a continuous basis and, when this is the case, the catalyst should preferably be in pelleted form. If this reaction is to be conducted on a batch basis, for example in an autoclave, the catalyst may be used in powdered form.

The reaction is conducted at a temperature of 100 to 200°C, and more preferably, 110 to 150°C, and at a pressure of 0.75 to 35 MPa, more preferably, 3.5 to 21 MPa.

The reactants are brought into contact with the hydrogenation catalyst in the presence of hydrogen. Suitably, hydrogen will be provided in an amount from the stoichiometric amount to a 1000 percent excess.

The diamino-polyoxyethylene and the formaldehyde remain in contact with the hydrogenation catalyst for a period of time sufficient to convert the diamino-polyoxyethylene substantially quantitatively and selectively into the corresponding tetramethyldiamino-polyoxyethylene. When the reaction is conducted on a batch basis, this can normally be accomplished in the useful reaction time of 1/2 hour to 30 hours.

When the reaction is conducted on a continuous basis, by continuously passing the diamino-polyoxyethylene and formaldehyde through a bed of pelleted catalyst, the reactants are suitably passed to the bed at the rate of 0.10 to 1.0 grams per hour of diamino-polyoxyethylene per cubic centimeter of catalyst and at the rate of 0.15 to 1.6 grams of formaldehyde per hour per cm³ of catalyst.

The formaldehyde is suitably charged to the reaction zone in solution in water or methanol. The formaldehyde solution will suitably contain from 10 to 55 wt. % of formaldehyde. For example, formalin (a 37 wt. % solution of formaldehyde and water) or a concentrated solution of formaldehyde and methanol containing about 55 wt. % of formaldehyde may be used.

The tetramethyldiamino-polyoxyethylene reaction product that is formed by the process of the present invention can be recovered from the reaction mixture with comparative ease by simple distillation.

### EXAMPLES

The present invention will be further illustrated by the following specific Examples.

The nickel, copper, chromium catalyst used in the Examples comprised about 75 mol % of nickel, about 23 mol % of copper and about 2 mol % of chromium, on an oxide-free basis.

### Example 1

A 600 cm³ tubular reactor filled with the nickel, copper, chromium catalyst was used. Feed No. 1 was triethylene glycol diamine and methanol in a 1:2 weight ratio; Feed No. 2 was 55% formaldehyde in methanol and the third feed was hydrogen. The reactor effluent was stripped at reduced pressure to remove the solvent. It was then analyzed by gas chromatography.

| Temperature | No. 1 Feed kg/h | No. 2 Feed kg/h | H₂ kg/h | Purity Hy GC Area % |
|---|---|---|---|---|
| 110°C | 0.53 | 0.29 | 99.9 | 98 |
| 120°C | 0.53 | 0.29 | 99.9 | 96 |

The above example shows that few byproducts are formed in this reaction. Purities above 90% were obtained without distillation.

### Example 2

The same procedure was used as in Example 1. The reaction temperature was 110°C. The crude product was first stripped at reduced pressure, and then distilled at 119-122°C and at 1.2 kPa. The purity by gas chromatography was 99.5%.

## Claims

1. A method for the preparation of a tetramethyldiamino-polyoxyethylene having the formula:
(I) (CH₃)₂N(CH₂)₂-O-[(CH₂)₂-O-]_{y}-(CH₂)₂N(CH₃)₂
which comprises reacting a diamino-polyoxyethylene having the formula:
(II) H₂N(CH₂)₂-O-[(CH₂)₂-O-]_{y}-(CH₂)₂NH₂
wherein y is 1 to 2, with a molar excess of formaldehyde in contact with a nickel or cobalt hydrogenation catalyst and in the presence of hydrogen at a temperature of 100 to 200°C and a pressure of 0.75 to 35.0 MPa.

2. A method according to Claim 1 characterised in that the nickel hydrogenation catalyst comprises, on an oxygen-free basis, 60 to 85 mol % of nickel, 14 to 37 mol % of copper and 1 to 3 mol % of chromium.

3. A method according to Claim 2 characterised in that the hydrogenation catalyst comprises, on an oxygen-free basis, 72 to 78 mol % of nickel, 20 to 25 mol % of copper and 1 to 3 mol % of chromium.

4. A method according to any one of Claims 1 to 3 characterised in that the formaldehyde is brought into contact with the hydrogenation catalyst in a solution containing 10 to 55 wt. % of formaldehyde, in a solvent selected from methanol and water.

5. A method according to Claim 4 characterised in that the formaldehyde solution contains 10 to 37 wt. % of formaldehyde in methanol.

6. A method according to any one of Claims 1 to 5 characterised in that the reaction is carried out in a continuous reactor containing a bed of the hydrogenation catalyst, the diamino-polyoxyethylene is passed through the catalyst bed at a rate of 0.1 to 1.0 grams per cubic centimeter of catalyst per hour, and the formaldehyde is passed through the catalyst bed at a rate of 0.15 to 1.6 grams of formaldehyde per cubic centimeter of catalyst per hour.
